# FASCICULE DE BREVET EUROPEEN

(11) **EP 4 003 607 B1**
(45) Date de publication et mention de la délivrance du brevet: **06.09.2023**
(21) Numéro de dépôt: 20757378.3
(22) Date de dépôt: 20.07.2020
(51) Int. Cl.: B05B 11/10, B05B 11/00, A61M 15/00, A61M 15/08

(54) **ENSEMBLE DE DISTRIBUTION COMPORTANT UN DISPOSITIF D'ACTIONNEMENT LATÉRAL POUR DISTRIBUTEUR DE PRODUIT FLUIDE**
SPENDEREINHEIT MIT EINER LATERALEN BETÄTIGUNGSVORRICHTUNG FÜR EINEN SPENDER EINES FLUIDPRODUKTS
DISPENSING ASSEMBLY WITH A LATERAL ACTUATING DEVICE FOR A FLUID PRODUCT DISPENSER

(30) Priorité: 26.07.2019 FR 1908524
(43) Date de publication de la demande: 01.06.2022
(73) Titulaire: APTAR France SAS, 27110 Le Neubourg (FR)
(72) Inventeur: ADAM, Fabien, 27930 Aviron (FR); GRAINE, Lila, 78650 Beynes (FR)
(74) Mandataire: CAPRI
(86) Numéro de dépôt international: PCT/FR2020/051301
(87) Numéro de publication internationale: WO 2021/019145

(56) Documents cités:
- EP-A1- 1 974 829
- WO-A1-03/103760
- US-A- 3 084 871
- US-A- 3 478 935

## Description

La présente invention concerne un ensemble de distribution comportant un dispositif d'actionnement latéral pour actionner un distributeur de produit fluide, notamment un dispositif de pulvérisation nasale d'un produit pharmaceutique.

Les distributeurs de produit fluide sont bien connus dans l'état de la technique. Ils comportent généralement un réservoir contenant le produit fluide sur lequel est assemblé un organe de distribution, par exemple une pompe ou une valve, qui est généralement actionné au moyen d'une tête de distribution pour distribuer de manière sélective le produit contenu à l'intérieur dudit réservoir. La tête de distribution comporte un orifice de distribution à travers lequel le produit va être pulvérisé, par exemple dans le nez de l'utilisateur dans le cas d'un dispositif de pulvérisation nasale. De nombreux dispositifs de ce type sont actionnés manuellement par l'utilisateur en déplaçant axialement l'un contre l'autre le réservoir et la tête de distribution, ce qui a pour effet d'actionner l'organe de distribution. Ce type de dispositif présente toutefois des inconvénients, notamment lorsque le dispositif est du type à pulvérisation nasale, car la force axiale exercée par l'utilisateur pour actionner le dispositif induit un risque de déplacement de la tête de distribution à l'intérieur de la narine de l'utilisateur, avec des risques de blessure et/ou de distribution incomplète ou inadéquate du produit au moment de l'actionnement.

Pour remédier à ce problème, des dispositifs d'actionnement latéral ont été proposés, comportant généralement un levier monté pivotant sur un corps et dont une partie interne est adaptée à coopérer avec l'un parmi la tête de distribution ou le réservoir pour déplacer cet élément contre l'autre et ainsi actionner l'organe de distribution. Un inconvénient de ce type de dispositifs d'actionnement latéral est le surcoût qu'il représente pour le distributeur de produit fluide. Un autre inconvénient est l'encombrement, notamment en direction latérale, du dispositif d'actionnement latéral.

Les documents US 3 084 871 A, EP1974829, US3478935, WO2014077839,
WO031 03760 et GB1531308 décrivent des dispositifs de l'état de la technique.

La présente invention a pour but de fournir un ensemble de distribution comportant un dispositif d'actionnement latéral qui ne reproduit pas les inconvénients susmentionnés.

La présente invention a aussi pour but de fournir un tel dispositif d'actionnement latéral qui qui puisse être utilisé avec plusieurs distributeurs de produit fluide, limitant ainsi son surcoût tout en garantissant un actionnement sûr et fiable du distributeur de produit fluide à chaque actionnement, sans risque de blessure pour l'utilisateur.

Plus particulièrement, la présente invention a pour but de fournir un ensemble de distribution comportant un dispositif d'actionnement latéral qui soit simple et peu coûteux à fabriquer et à assembler.

La présente invention a donc pour objet un ensemble de distribution, comportant un distributeur de produit fluide, tel qu'un dispositif de distribution nasale, dans lequel ledit distributeur de produit fluide comporte un réservoir pourvu d'un fond, un organe de distribution, tel qu'une pompe ou une valve, monté sur ledit réservoir et une tête de distribution incorporant un orifice de distribution, ledit ensemble de distribution comportant un dispositif d'actionnement latéral comportant un corps et un élément d'actionnement latéral monté pivotant sur ledit corps autour d'un axe de pivotement entre une position de repos et une position d'actionnement, ledit élément d'actionnement comportant une partie d'appui, sur laquelle l'utilisateur appuie lors de l'actionnement, et une partie de poussée adaptée à coopérer avec ledit distributeur de produit fluide pour réaliser son actionnement, ledit axe de pivotement étant disposé entre ladite partie d'appui et ladite partie de poussée, ledit élément d'actionnement latéral ayant une forme de S ou de S inversé, ladite partie d'appui étant disposée à proximité de ladite tête de distribution et ladite partie de poussée étant disposée sous ledit fond dudit réservoir, ledit élément d'actionnement comportant deux branches identiques parallèles reliées l'une à l'autre par lesdites parties d'appui et de poussée, lesdites branches comportant une première courbure au niveau de leur jonction à ladite partie d'appui et une seconde courbure, opposée à ladite première courbure, au niveau de leur jonction à ladite partie de poussée.

Avantageusement, lesdites première et seconde courbures sont tournées vers l'intérieur dudit corps.

Avantageusement, ledit axe de pivotement est disposé plus proche de ladite partie de poussée que de ladite partie d'appui.

Avantageusement, un capot de protection amovible est monté sur ledit corps.

Avantageusement, ledit capot de protection comporte une extension axiale coopérant avec ledit élément d'actionnement pour empêcher son pivotement.

Avantageusement, ledit axe de pivotement s'étend radialement à l'extérieur dudit réservoir.

En variante, ledit axe de pivotement est disposé sensiblement sécant à l'axe central longitudinal dudit réservoir.

Ces caractéristiques et avantages et d'autres de la présente invention apparaîtront plus clairement au cours de la description détaillée suivante de plusieurs modes et variantes de réalisation, faite en référence aux dessins joints, donnés à titre d'exemples non limitatifs, sur lesquels :
la figure 1 représente une vue schématique de côté d'un ensemble selon un premier mode de réalisation avantageux, avant retrait du capot de protection ;
[les figures 2 et 3 représentent des vues schématiques de côté de l'ensemble de la figure 1, respectivement en position de repos et en position actionnée ;
les figures 4 et 5 représentent des vues schématiques partielles en perspective selon deux angles de vue différents d'un élément d'actionnement latéral selon une variante avantageuse associé à un distributeur de produit fluide;
la figure 6 représente une vue schématique de côté d'un ensemble selon un second mode de réalisation avantageux, avant retrait du capot de protection ; et
les figures 7 et 8 représentent des vues schématiques de côté de l'ensemble de la figure 6, respectivement en position de repos et en position actionnée.

Dans la description ci-après, les termes "supérieur", "inférieur", "haut" et "bas" se réfèrent à la position droite du dispositif représentée sur les figures. Les termes "axial", "latéral" et "radial" se réfèrent à l'axe central longitudinal du distributeur.

En se référant aux figures, qui illustrent deux modes de réalisation avantageux, il est représenté un ensemble de distribution comportant d'une part un dispositif d'actionnement latéral et d'autre part un distributeur de produit fluide, notamment un dispositif de pulvérisation nasale.

Le distributeur de produit fluide comporte un réservoir 10 qui contient du produit fluide, notamment du produit pharmaceutique destiné à être pulvérisé dans le nez d'un utilisateur. Un organe de distribution 20, tel qu'une pompe ou une valve, est assemblé sur ledit réservoir 10, au moyen d'une bague de fixation (non représentée) qui peut être sertissable, encliquetable ou vissable. L'organe de distribution 20, qui n'est pas représenté en détail sur les figures, comporte classiquement un corps d'organe de distribution, tel qu'un corps de pompe ou un corps de valve, et un organe mobile, tel qu'une tige de piston ou une soupape de valve, qui est monté coulissant axialement dans ledit corps d'organe de distribution. De manière classique, l'organe mobile est enfoncé à l'intérieur du corps d'organe de distribution, pour actionner ledit organe de distribution. Une tête de distribution 30 est assemblée sur ledit organe de distribution 20, ladite tête de distribution 30 incorporant un orifice de distribution 31 à travers lequel le produit fluide est distribué.

Un dispositif d'actionnement latéral est prévu pour être utilisable avec plusieurs distributeurs de produit fluide. Ainsi, le distributeur de produit fluide est monté de manière amovible dans ledit dispositif d'actionnement latéral, et lorsque le réservoir 10 est vide, le distributeur est retiré et remplacé par un autre distributeur comportant un réservoir 10 plein.

Le dispositif d'actionnement latéral comporte un corps 1 adapté à recevoir ledit distributeur. Le corps 1 peut être réalisé en une seule partie monobloc, ou il peut comporter deux ou plusieurs parties fixées l'une à l'autre lors de l'assemblage du distributeur dans le corps. Dans la suite de la description, il sera fait référence au corps 1, étant entendu que ceci englobe les deux variantes ci-dessus.

Avantageusement, le corps 1 est ouvert axialement vers le haut pour permettre l'insertion et le retrait du distributeur à travers cette ouverture axiale. Avec un corps 1 en plusieurs parties assemblées, on pourrait envisager un sens d'assemblage différent.

Un capot de protection amovible 2 est assemblé sur le corps 1 pour protéger l'orifice de distribution 31 entre deux actionnements. Ce capot 2 peut comporter une extension 3 qui bloque l'actionnement du dispositif d'actionnement latéral lorsque le capot 2 est en place sur le corps 1. Ceci permet d'empêcher tout actionnement accidentel, par exemple pendant le transport.

Le dispositif d'actionnement latéral comporte un élément d'actionnement latéral 40 solidaire du corps 1 et adapté à coopérer avec le réservoir 10. Cet élément d'actionnement latéral 40 comporte une partie d'appui 41 sur laquelle l'utilisateur appuie pour actionner le distributeur, et une partie de poussée 42 coopérant avec le fond 11 du réservoir 10. L'élément d'actionnement latéral 40 est monté pivotant sur le corps 1 autour d'un axe 45.

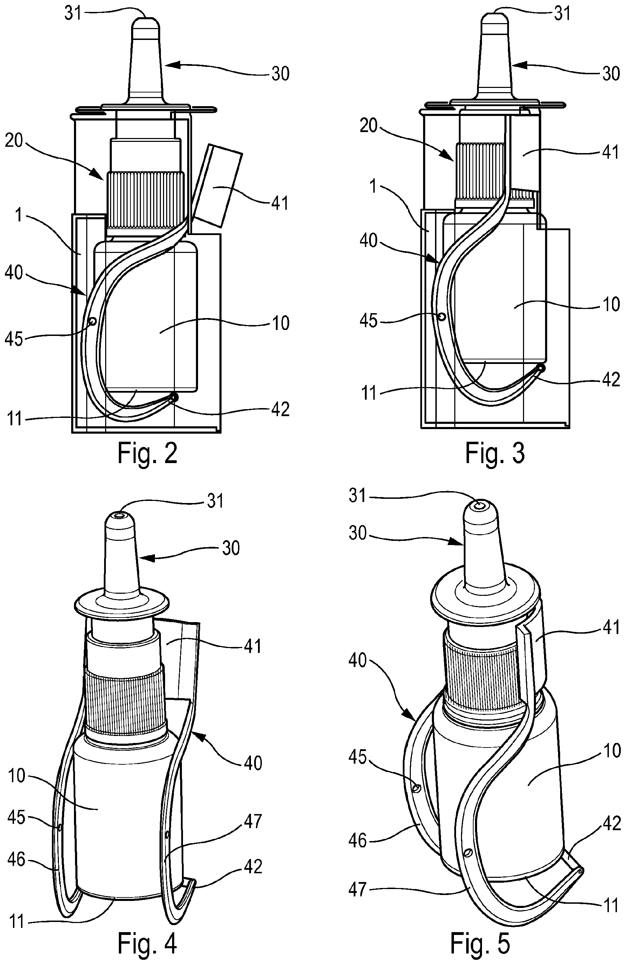

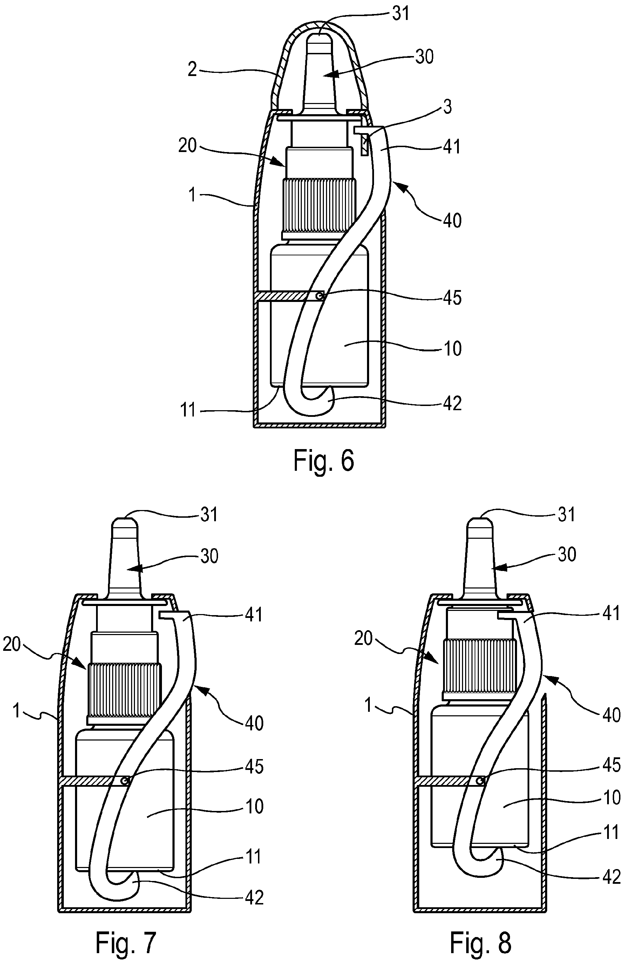

Selon l'invention, l'élément d'actionnement latéral 40 a environ une forme de S ou de S inversé, cette seconde variante étant celle représentée sur les dessins. Ainsi, la partie d'appui 41 est disposée à proximité de la tête de distribution 30 et la partie de poussée 42 est disposée sous le fond 11 du réservoir 10. L'axe de pivotement 45 est disposé entre ladite partie d'appui 41 et ladite partie de poussée 42. Cette forme en S ou S inversé de l'élément d'actionnement 40 permet de limiter son encombrement latéral, de sorte qu'en position de repos, visible sur les figures 2 et 7, la partie d'appui 41 ne s'étend que peu radialement ou latéralement hors du corps 10. De préférence, l'axe de pivotement 45 est disposé plus proche de la partie de poussée 42 que de la partie d'appui 41. Ceci permet notamment d'optimiser l'effet de levier et donc de rendre l'actionnement plus aisé pour l'utilisateur.

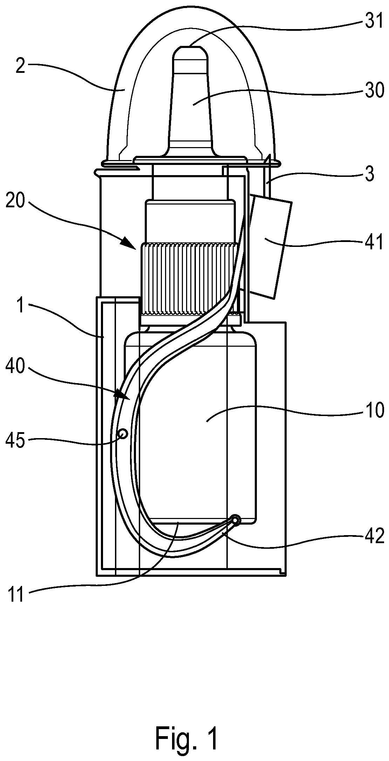

Comme visible sur les figures 1 et 6, lorsque le capot 2 est fixé au corps 1, l'extension axiale 3 du capot 2 coopère avec la partie d'appui 41 de l'élément d'actionnement 40 pour empêcher le pivotement dudit élément d'actionnement.

De préférence, l'élément d'actionnement 40 comporte deux branches identiques parallèles 46, 47 reliées l'une à l'autre par les partie d'appui 41 et de poussée 42, comme visible notamment sur les figures 4 et 5. La première branche 46 s'étend d'un côté du réservoir 10 et la seconde branche 47 s'étend de l'autre côté du réservoir 10. Ces branches 46, 47 comportent une première courbure au niveau de leur jonction à la partie d'appui 41 et une seconde courbure, opposée à la première courbure, au niveau de leur jonction à la partie de poussée 42. Comme visible sur les figures, ces deux courbures opposées sont toutes deux tournées vers l'intérieur du corps 1.

Dans le mode de réalisation des figures 1 à 5, l'axe de pivotement 45 s'étend radialement à l'extérieur du réservoir 10 lorsqu'un distributeur est assemblé dans le dispositif d'actionnement latéral. Dans le mode de réalisation des figures 6 à 8, l'axe de pivotement 45 est disposé sensiblement sécant à l'axe central longitudinal du réservoir 10.

En fonctionnement, l'utilisateur appuie sur la partie d'appui 41 de l'élément d'actionnement 40 pour le faire pivoter par rapport au corps 1 autour de l'axe 45. Ce pivotement de l'élément d'actionnement 40 provoque le déplacement axial vers le haut de la partie de poussée 42 et donc du réservoir 10 et ainsi l'actionnement de l'organe de distribution 20.

L'élément d'actionnement 40 peut comporter un élément élastique (non représenté), tel qu'une lame élastique ou un ressort, qui sollicite élastiquement ledit élément d'actionnement 40 vers sa position de repos. En variante, l'élément d'actionnement 40 peut être ramené vers sa position de repos par le réservoir 10 lorsqu'il revient vers sa position de repos après chaque actionnement sous l'effet du ressort de rappel de l'organe de distribution 20.

Bien que l'invention ait été décrite en références à des modes de réalisations regroupant plusieurs des aspects décrits ci-dessus, il est entendu que diverses modifications sont envisageables pour l'homme du métier sans sortir du cadre de la présente invention tel que défini par les revendications annexées.

## Revendications

1. Ensemble de distribution, comportant un distributeur de produit fluide, tel qu'un dispositif de distribution nasale, dans lequel ledit distributeur de produit fluide comporte un réservoir (10) pourvu d'un fond (11), un organe de distribution (20), tel qu'une pompe ou une valve, monté sur ledit réservoir (10) et une tête de distribution (30) incorporant un orifice de distribution (31), ledit ensemble de distribution comportant un dispositif d'actionnement latéral comportant un corps (1) et un élément d'actionnement latéral (40) monté pivotant sur ledit corps (1) autour d'un axe de pivotement (45) entre une position de repos et une position d'actionnement, ledit élément d'actionnement (40) comportant une partie d'appui (41), sur laquelle l'utilisateur appuie lors de l'actionnement, et une partie de poussée (42) adaptée à coopérer avec ledit distributeur de produit fluide pour réaliser son actionnement, ledit axe de pivotement (45) étant disposé entre ladite partie d'appui (41) et ladite partie de poussée (42), ledit élément d'actionnement latéral (40) ayant une forme de S ou de S inversé, ladite partie d'appui (41) étant disposée à proximité de ladite tête de distribution (30) et ladite partie de poussée (42) étant disposée sous ledit fond (11) dudit réservoir (10), ledit élément d'actionnement (40) comportant deux branches identiques parallèles (46, 47) reliées l'une à l'autre par lesdites parties d'appui (41) et de poussée (42), **caractérisé en ce que** lesdites branches (46, 47) comportent une première courbure au niveau de leur jonction à ladite partie d'appui (41) et une seconde courbure, opposée à ladite première courbure, au niveau de leur jonction à ladite partie de poussée (42).

2. Ensemble selon la revendication 1, dans lequel lesdites première et seconde courbures sont tournées vers l'intérieur dudit corps (1).

3. Ensemble selon la revendication 1 ou 2, dans lequel ledit axe de pivotement (45) est disposé plus proche de ladite partie de poussée (42) que de ladite partie d'appui (41).

4. Ensemble selon l'une quelconque des revendications précédentes, dans lequel un capot de protection amovible (2) est monté sur ledit corps (1).

5. Ensemble selon la revendication 4, dans lequel ledit capot de protection (2) comporte une extension axiale (3) coopérant avec ledit élément d'actionnement (40) pour empêcher son pivotement.

6. Ensemble selon l'une quelconque des revendications précédentes, dans lequel ledit axe de pivotement (45) s'étend radialement à l'extérieur dudit réservoir (10).

7. Ensemble selon l'une quelconque des revendications 1 à 5, dans lequel ledit axe de pivotement (45) est disposé sensiblement sécant à l'axe central longitudinal dudit réservoir (10).

## Patentansprüche

1. Spendereinheit aufweisend einen Fluidproduktspender wie etwa eine Vorrichtung zur nasalen Verabreichung, wobei der Fluidproduktspender einen Vorratsbehälter (10), der mit einem Boden (11) versehen ist, ein Ausgabeorgan (20) wie etwa eine Pumpe oder ein Ventil, das auf dem Vorratsbehälter (10) angebracht ist, und einen Ausgabekopf (30) aufweist, der eine Ausgabeöffnung (31) enthält, wobei die Spendereinheit eine seitliche Betätigungsvorrichtung aufweist, die einen Körper (1) und ein seitliches Betätigungselement (40) aufweist, das auf dem Körper (1) um eine Schwenkachse (45) zwischen einer Ruheposition und einer Betätigungsposition schwenkbar angebracht ist, wobei das Betätigungselement (40) einen Drückabschnitt (41), auf den der Benutzer bei der Betätigung drückt, und einen Schubabschnitt (42) aufweist, der geeignet ist, mit dem Fluidproduktspender zusammenzuwirken, um dessen Betätigung auszuführen, wobei die Schwenkachse (45) zwischen dem Drückabschnitt (41) und dem Schubabschnitt (42) angeordnet ist, wobei das seitliche Betätigungselement (40) eine S-Form oder umgekehrte S-Form aufweist, wobei der Drückabschnitt (41) in der Nähe des Ausgabekopfes (30) angeordnet ist und der Schubabschnitt (42) unter dem Boden (11) des Vorratsbehälters (10) angeordnet ist, wobei das Betätigungselement (40) zwei parallele, identische Bügel (46, 47) aufweist, die durch den Drück- (41) und den Schubabschnitt (42) miteinander verbunden sind, **dadurch gekennzeichnet, dass** die Bügel (46, 47) eine erste Krümmung an ihrer Anbindung an den Drückabschnitt (41) und eine zweite Krümmung, die der ersten Krümmung entgegengesetzt ist, an ihrer Anbindung an den Schubabschnitt (42) aufweisen.

2. Einheit nach Anspruch 1, wobei die erste und zweite Krümmung zum Inneren des Körpers (1) hin gewandt sind.

3. Einheit nach Anspruch 1 oder 2, wobei die Schwenkachse (45) näher an dem Schubabschnitt (42) als an dem Drückabschnitt (41) angeordnet ist.

4. Einheit nach einem der vorhergehenden Ansprüche, wobei eine abnehmbare Schutzkappe (2) auf dem Körper (1) angebracht ist.

5. Einheit nach Anspruch 4, wobei die Schutzkappe (2) einen axialen Fortsatz (3) aufweist, der mit dem Betätigungselement (40) zusammenwirkt, um dessen Schwenken zu verhindern.

6. Einheit nach einem der vorhergehenden Ansprüche, wobei sich die Schwenkachse (45) radial außerhalb des Vorratsbehälters (10) erstreckt.

7. Einheit nach einem der Ansprüche 1 bis 5, wobei die Schwenkachse (45) im Wesentlichen sekantial zur Längsmittelachse des Vorratsbehälters (10) angeordnet ist.

## Claims

1. Dispensing assembly, comprising a fluid product dispenser, such as a nasal dispensing device, wherein said fluid product dispenser comprises a reservoir (10) provided with a bottom (11), a dispensing member (20), such as a pump or a valve, mounted on said reservoir (10) and a dispensing head (30) incorporating a dispensing orifice (31), said dispensing assembly comprising a lateral actuation device comprising a body (1) and a lateral actuation element (40) mounted on said body (1) to pivot about a pivot axis (45) between a rest position and an actuation position, said actuation element (40) comprising a bearing part (41), on which the user presses during actuation, and a pushing part (42) adapted to cooperate with said fluid product dispenser to effect actuation thereof, said pivot axis (45) being arranged between said bearing part (41) and said pushing part (42), said lateral actuation element (40) having a shape of S or inverted S, said bearing part (41) being arranged close to said dispensing head (30) and said pushing part (42) being arranged under said bottom (11) of said reservoir (10), said actuation element (40) comprising two parallel identical branches (46, 47) connected to one another by said bearing (41) and pushing (42) parts, **characterized in that** said branches (46, 47) comprise a first curvature at their joining to said bearing part (41) and a second curvature, opposite said first curvature, at their joining to said pushing part (42).

2. Assembly according to claim 1, wherein said first and second curvatures are facing towards the inside of said body (1).

3. Assembly according to claim 1 or 2, wherein said pivot axis (45) is arranged closer to said pushing part (42) than said bearing part (41).

4. Assembly according to any one of the preceding claims, wherein a removable protective cap (2) is mounted on said body (1).

5. Assembly according to claim 4, wherein said protective cap (2) comprises an axial extension (3) cooperating with said actuation element (40) to prevent its pivoting.

6. Assembly according to any one of the preceding claims, wherein said pivot axis (45) extends radially outside said reservoir (10).

7. Assembly according to any one of claims 1 to 5, wherein said pivot axis (45) is arranged substantially secant to the longitudinal central axis of said reservoir (10).
